# EUROPEAN PATENT SPECIFICATION

(11) **EP 4 555 322 B1**
(45) Date of publication and mention of the grant of the patent: **22.10.2025**
(21) Application number: 23782299.4
(22) Date of filing: 20.09.2023
(51) Int. Cl.: G01N 33/53

(54) **METHOD FOR FIXING PRIMARY ANTIBODIES TO A BIOLOGICAL SAMPLE**
VERFAHREN ZUR FIXIERUNG PRIMÄRER ANTIKÖRPER AN EINE BIOLOGISCHE PROBE
PROCÉDÉ DE FIXATION D'ANTICORPS PRIMAIRES À UN ÉCHANTILLON BIOLOGIQUE

(30) Priority: 27.09.2022 US 202263410438 P; 10.10.2022 US 202263414883 P
(43) Date of publication of application: 21.05.2025
(73) Proprietor: PIXELGEN TECHNOLOGIES AB, 171 65 Solna (SE)
(72) Inventor: FREDRIKSSON, Simon, 171 65 Solna (SE)
(74) Representative: Williams Powell
(86) International application number: PCT/IB2023/059313
(87) International publication number: WO 2024/069322

(56) References cited:
- WO-A1-2016/118065
- US-A1- 2013 184 184
- DI WU ET AL: "Profiling surface proteins on individual exosomes using a proximity barcoding assay", NATURE COMMUNICATIONS, vol. 10, no. 1, 26 August 2019 (2019-08-26), XP055767129, DOI: 10.1038/s41467-019-11486-1
- LAMVIK JON ET AL: "NONLABELED SECONDARY ANTIBODIES AUGMENT/MAINTAIN THE BINDING OF PRIMARY, SPECIFIC ANTIBODIES TO CELL MEMBRANE ANTIGENS", CYTOMETRY, ALAN LISS, NEW YORK, US, vol. 45, 18 January 2001 (2001-01-18), pages 187 - 193, XP002441130, ISSN: 0196-4763, DOI: 10.1002/1097-0320(20011101)45:3<187::AID-CYTO1162>3.0.CO;2-7

## Description

### CROSS-REFERENCING

This application claims the benefit of provisional application serial nos. 63/410,438, filed on September 27, 2022, and 63/414,883, filed on October 10, 2022.

### BACKGROUND

The analysis of biomolecules in cells and tissues is often performed by binding of primary target specific antibodies and detecting these with fluorescence in a microscope. Some readout techniques of these reagents use a primary antibody coupled to a DNA sequence. These DNA sequences can be used for identification and localization of the binding site of the primary antibody in and on the cell/tissue sample. Often, another oligonucleotide coupled to a fluorophore hybridizes to the DNA coupled to the primary antibody. This way multiple primary antibodies with unique DNA-tags can be serially analyzed using sequential hybridization and fluorescence detection. DNA tagged antibodies are particularly useful in multiplexed detection of many target proteins.

One problem with such assays is that the primary antibodies need to remain bound to their target protein throughout the complex DNA detection process. If the DNA detection process has harsh conditions such as elevated temperatures, organic solvents, high salt concentrations the primary antibodies may release from the sample by loss of target binding, resulting in significant loss of signal. In addition, some DNA detection technologies require the use of enzymes that use DNA as a substrate (e.g., DNA polymerases, ligases, etc.) to enable the detection. These enzymes require some time and temperature elevation which can severely impact the stability of the primary antibody binding.

One solution to this problem is to covalently fix the primary antibody-oligonucleotide conjugate to the sample using a crosslinking chemical fixative. In these methods, the primary antibodies are covalently coupled to the sample. Paraformaldehyde is one such x-linking reagent, another is a divalent NHS-ester that randomly couples primary amines to each other. However, these chemical crosslinking agents can potentially affect the integrity of the DNA coupled to the antibodies, thereby lowering the efficiency of signal generation. Alternative solutions are therefore needed.

Document WO2016/118065A1, discloses a method for preparing a biological sample for use in an immunolabeling process. Document Di WU ET AL: "Profiling surface proteins on individual exosomes using a proximity barcoding assay",NATURE COMMUNICATIONS, vol. 10, no. 1, 26 August 2019 (2019-08-26), discloses a method for profiling surface proteins through a proximity bar code assay.

### SUMMARY

The invention is defined by the apended claims.

Provided herein is an alternative to chemical crosslinking for stabilizing the binding of primary antibodies bound to their target protein in a biological sample. The method finds particular use in stabilizing the binding of an antibody-oligonucleotide conjugate (i.e., a conjugate comprising an antibody and an oligonucleotide that is linked to the antibody). In these embodiments, the method involves labeling the sample with one or more antibody-oligonucleotide conjugates and then stabilizing the binding of the conjugates to the sample using a secondary antibody. In these methods, the antibody-oligonucleotide conjugates are held in place throughout the DNA detection procedures by the secondary antibodies, which are unlabeled (i.e., not detectably labeled or conjugated to an affinity tag such as biotin).

Without being wished to any particular theory, it is thought that the arms of the secondary bind to different molecules of antibody-oligonucleotide conjugates, effectively linking the conjugates together and stabilizing the multicomponent complex.

A method for labeling a biological sample is provided. In some embodiments, the method may comprise: labeling the biological sample with one or more primary antibody-oligonucleotide conjugates to produce a labeled sample, incubating the labeled sample with a secondary antibody that recognizes the primary antibody of the primary antibody-oligonucleotide conjugates, thereby cross-linking the primary antibody and producing a cross-linked sample, performing one or more molecular reactions on the oligonucleotide of the primary antibody-oligonucleotide conjugates to produce a nucleic acid product; and detecting the nucleic acid product. The method may be done in the absence of any crosslinking steps that are done after the sample has been labeled with one or more primary antibody-oligonucleotide conjugates (i.e., in the absence of any post-labeling treatment with an amine-to-amine crosslinker (e.g. formaldehyde, di-N-hydroxysuccinimide esters or another reagents of similar action) in some cases. However, in the proposed method, the sample may have been crosslinked by standard fixatives (e.g., PFA) for sample preservation before it is labeled with the antibody.

### BRIEF DESCRIPTION OF THE FIGURES

The skilled artisan will understand that the drawings, described below, are for illustration purposes only. The drawings are not intended to limit the scope of the present teachings in any way.
Fig. 1 illustrates certain principles of an embodiment of the method.
Fig. 2 is an image of a gel demonstrating that the addition of secondary antibodies increases binding stability of oligonucleotide-antibody conjugates to a sample.
Fig. 3 shows a comparison between a monoclonal and a polyclonal cross-linking secondary antibody showing either the average number of Antibody Oligonucleotide Conjugates (AOCs) detected per single cell (left), or the average number of Pixel As per single cell. Both metrics show higher efficiency for the polyclonal secondary antibody compared to the monoclonal secondary antibody due to primary AOCs being more efficiently immobilized/linked to the sample.

### DEFINITIONS

Unless defined otherwise herein, all technical and scientific terms used herein have the same meaning as commonly understood by one of ordinary skill in the art to which this invention belongs. Although any methods and materials similar or equivalent to those described herein can be used in the practice or testing of the present invention, the preferred methods and materials are described.

Numeric ranges are inclusive of the numbers defining the range. Unless otherwise indicated, nucleic acids are written left to right in 5' to 3' orientation; amino acid sequences are written left to right in amino to carboxy orientation, respectively.

The headings provided herein are not limitations of the various aspects or embodiments of the invention. Accordingly, the terms defined immediately below are more fully defined by reference to the specification as a whole.

Unless defined otherwise, all technical and scientific terms used herein have the same meaning as commonly understood by one of ordinary skill in the art to which this invention belongs. Singleton, et al., DICTIONARY OF MICROBIOLOGY AND MOLECULAR BIOLOGY, 2D ED., John Wiley and Sons, New York (1994), and Hale & Markham, THE HARPER COLLINS DICTIONARY OF BIOLOGY, Harper Perennial, N.Y. (1991) provide one of skill with the general meaning of many of the terms used herein. Still, certain terms are defined below for the sake of clarity and ease of reference.

As used herein, the term "labeling" refers to binding antibodies to specific sites in a sample (i.e., sites that contain an antigen for the antibodies being used). Methods for binding antibodies and aptamers to sites in the sample are well known. As would be apparent, labeling requires incubating the sample and conjugates under conditions (which conditions include a period of time, a temperature, an appropriate binding buffer and a wash) that are suitable for specific binding of the antibodies to molecules (e.g., epitopes) in the sample sample.

As used herein, the term "primary antibody" refers to an antibody that recognizes antigens that are native to a biological sample. Such antibodies are typically monoclonal antibodies, e.g., mouse or rabbit monoclonal antibodies that recognize human antigens.

As used herein, the term "antibody-oligonucleotide conjugate" refers to a conjugate between an antibody and an oligonucleotide.

As used herein, the term "secondary antibody" refers to antibody that is anti-primary antibody. Secondary antibodies are produced by immunizing a second species of animal with antibodies from the species used to produce the primary antibody. Illustrated by example, if the primary antibody is rabbit or mouse, then the secondary antibody can be goat anti-rabbit or goat anti-mouse, produced by immunizing a goat with rabbit or mice polyclonal antiserum. The secondary antibodies used in this method are always multivalent. IgG can be readily used. However, IgM could be used too.

The term "oligonucleotide" as used herein denotes a single-stranded multimer of nucleotides of from about 2 to 200 nucleotides, up to 500 nucleotides in length. Oligonucleotides may be synthetic or may be made enzymatically, and, in some embodiments, are 30 to 150 nucleotides in length. Oligonucleotides may contain ribonucleotide monomers (i.e., may be oligoribonucleotides) or deoxyribonucleotide monomers. An oligonucleotide may be 10 to 20, 21 to 30, 31 to 40, 41 to 50, 51to 60, 61 to 70, 71 to 80, 80 to 100, 100 to 150 or 150 to 200 nucleotides in length, for example.

The terms "plurality", "set" and "population" are used interchangeably to refer to something that contains at least 2 members. In certain cases, a plurality may have at least 10, at least 100, at least 1000, at least 10,000, or at least 100,000 members.

The term "sequencing", as used herein, refers to a method by which the identity of at least 10 consecutive nucleotides (e.g., the identity of at least 20, at least 50, at least 100 or at least 200 or more consecutive nucleotides) of a polynucleotide are obtained.

Other definitions of terms may appear throughout the specification.

### DETAILED DESCRIPTION

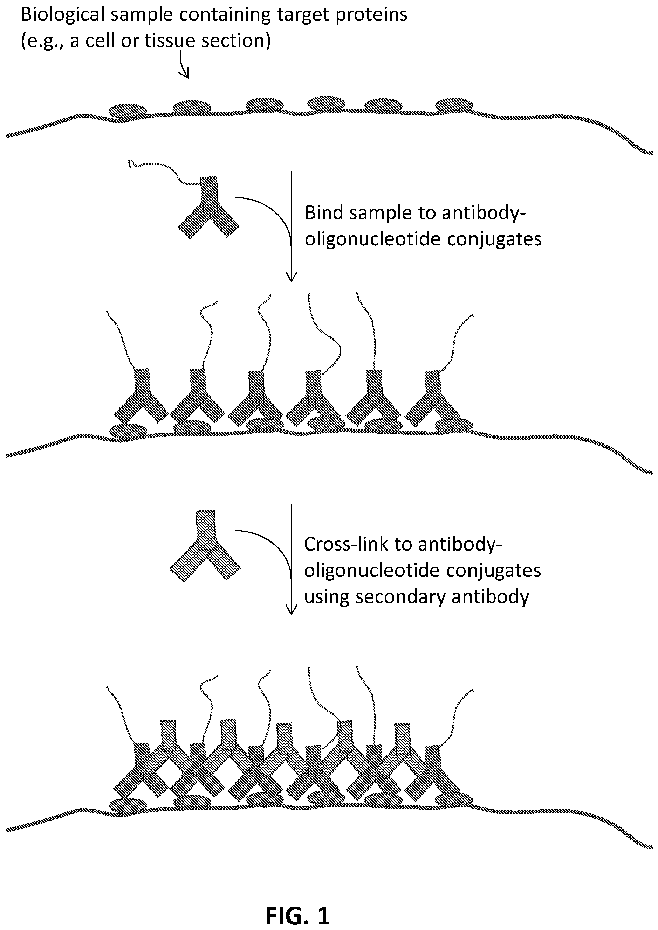

As summarized above, a method for labeling a biological sample is provided. In some embodiments, the method may comprise: labeling the biological sample with a one or more primary antibody-oligonucleotide conjugates to produce a labeled sample, incubating the labeled sample with a secondary antibody that recognizes the primary antibody of the primary antibody-oligonucleotide conjugates, thereby cross-linking the primary antibody and producing a cross-linked sample, performing one or more molecular reactions on the oligonucleotide of the primary antibody-oligonucleotide conjugates to produce a nucleic acid product; and detecting the nucleic acid product. Some principles of the method are illustrated in Fig. 1.

In some embodiments, the molecular reactions may comprise exposing the primary antibody-oligonucleotide conjugates to one or more disassociating conditions while they are bound to the sample (i.e., the primary antibody-oligonucleotide conjugates), wherein the disassociating conditions comprise one or more reactions, hybridizations, washes and/or treatments in at least 150 mM salt or an equivalent thereof at a temperature of at least 37 °C for at least 1 minute.

In any embodiment, the disassociating conditions can comprise one or more reactions, hybridizations, washes and/or treatments in at least 150 mM salt (e.g., 150 mM to 1 M NaCl or 250 mM to 500 mM NaCl, without or without a divalent cation, e.g., at least Mg₂Cl, e.g., or 5 mM to 100 mM Mg₂Cl ) at a temperature of at least 37 °C (e.g., a temperature in the range of 37 °C to 70 °C or 40 °C to 65 °C) for at least 1 minute (e.g., a time period in the range of 10 mins to 2 hr). For example, in any embodiment, the molecular reactions may comprise one, two, three or four reactions, hybridization, washes and/or treatments in 250 mM to 500 mM NaCl (or an equivalent thereof), 5 mM to 20 mM Mg₂Cl at a temperature of in the range of 40 °C to 65 °C, for a time period in the range of 10 mins to 2 hr. Proteins often dissociate under such conditions.

In some embodiments, any of the disassociating conditions may alternatively or in addition comprise exposing the bound conjugates to a chemical denaturant, e.g., formamide, DMSO, ethanol, methanol, urea and/or a chaotropic agent (e.g., guanidinium chloride or the like), at a concentration in the range of 5-50% for at least 1 minute, e.g., 5 mins to 1 hr. Depending on the denaturant and other parameters, the concentration of the denaturant may be higher, e.g., 70% to 90% (e.g., for DMSO or formamide).

In any embodiment, the disassociating conditions may comprise subjecting the antibodies to a physical shearing stress, e.g., by tethering to particle selected from a rolling circle amplification (RCA) product or a bead. In these embodiments, the oligonucleotide of a conjugate may be directly connected to a particle or indirectly connected to the particle via base-pairing (e.g., at least 15, at least 20 or at least 30 base pairs). RCA products are typically to be in excess of 1,000 kDa or 1,000 kDa and, as such, tethering to such particles will 'tug' at the primary antibodies and create a shearing force. The present method is believed to assist in holding the primary antibodies onto the cell, even when the antibodies are subjected to a physical shearing force. Rolling circle amplification or RCA for short is an isothermal amplification that generates linear concatemerized copies of a circular nucleic acid template using a strand-displacing polymerase. RCA is well known in the molecular biology arts and is described in a variety of publications including, but not limited to Lizardi et al (Nat. Genet. 1998 19:225-232), Schweitzer et al (Proc. Natl. Acad. Sci. 2000 97:10113-10119), Wiltshire et al (Clin. Chem. 2000 46:1990-1993) and Schweitzer et al (Curr. Opin. Biotech 2001 12:21-27), which are incorporated by reference herein. Rolling circle amplification products are the concatamerized products of a rolling circle amplification reaction. These molecules can contain hundreds or thousands of copies of the template. In this method, the conjugate contains an antibody that is non-covalently (e.g., via a streptavidin/biotin interaction) or covalently (e.g., via a click reaction or the like) linked to an oligonucleotide in a way that the antibody can still bind to its target. The nucleic acid and the antibody may be linked via a number of different methods, including those that use maleimide or halogen-containing group, which are cysteine-reactive. The antibody and the oligonucleotide may be linked at, proximal to or at the 5' end of the oligonucleotide, proximal to or at the 3' end of the oligonucleotide, or anywhere in-between. In some embodiments, the oligonucleotide contains a sequence that identifies the target to which the binding agent binds. For example, the oligonucleotide contains an antibody identifier sequence that identifies the antibody name or the target (e.g., epitope) to which the antibody binds. Oligonucleotides may be linked to capture agents using any convenient method (see, e.g., Gong et al., Bioconjugate Chem. 2016 27: 217-225 and Kazane et al. Proc Natl Acad Sci 2012 109: 3731-3736). As noted above, the sequence of the oligonucleotide that is conjugated to an antibody may uniquely identify the epitope or sequence to which the antibody binds. For example, if the method is performed using 10 different antibodies, then each antibody may be tethered to a different oligonucleotide that contains a sequence that identifies the target to which the antibody binds. This feature allows the method to be multiplexed and, in some embodiments, at least 5, at least 10, at least 20 or at least 50 different antibodies that bind to different targets (which may be on the surface of a cell or in a tissue sample) can be used in the method. Each antibody may conjugated to a different antibody identification sequence, and the antibody identification sequences allows the binding events for a particular antibody to be identified. In some embodiments, the labeling step of the method may comprises *en masse* labeling the biological sample with a plurality of primary antibody-oligonucleotide conjugates that recognize different targets in or on the sample (where the term "en masse" is intended to mean at the same time). For example, the sample may be labeled with at least 10 or at least 20 and up to 50 or 100 or more antibodies, each linked to a different oligonucleotide that identifies the antibody to which it is conjugated. In these embodiments, the detecting step may comprises sequencing at least the antibody identifier sequences in the nucleic acid reaction product, thereby identifying the antibodies that are bound to the sample.

The molecular reaction may be, for example, a hybridization, a ligation, a primer extension, or a gap-fill ligation, or any combination thereof. For example, the molecular reaction may comprises a hybridization with a nucleic acid followed by a primer extension and/or gap-fill ligation reaction using the either the oligonucleotide or the nucleic acid as a template. In other embodiments, the molecular reaction of may be a ligation, e.g., a splinted ligation. In some embodiments, the molecular reaction of (c) may be a proximity assay, e.g., proximity ligation reaction (e.g., PLA) or a proximity extension reaction (e.g., PEA). In any embodiment, the method may be used in the types of spatial assays described in PCT/IB2022/052862, filed on March 29, 2022 and entitled Spatial Mapping by Serial Primer Extension, the descriptions of which assays are incorporated by reference herein.

In any embodiment, at least part of step (c) (e.g., part or all of step (c)) may be done in the presence of the additional secondary antibodies, where the term 'additional' means that the secondary antibodies are added separately to the secondary antibodies that are already bound to the sample in step (a). In other words, a solution containing the additional secondary antibodies is added to the sample after step (a), immediately before or during the molecular reactions, where the additional secondary antibodies are present during the one or more molecular reactions. In these embodiments, the presence of the secondary antibodies may prevent re-binding of any primary antibody-oligonucleotide conjugates that have dissociated during the one or more molecular reactions, thereby reducing background.

The present methods do not rely on any labeled antibodies and, as such, do not use labeled tertiary antibodies (that bind to the secondary antibodies) or any other labeling system that binds to or interacts with the secondary antibodies. Rather, the oligonucleotides used in the method and the output is a sequence.

In any embodiment, the detecting may be done by sequencing the product, or an amplification product thereof. In these embodiments, the oligonucleotide that is conjugated to the antibody may have a site for a PCR primer in addition to the antibody identifier sequence thereby allowing the product to be amplified before sequencing. As would be apparent, in these embodiments, the additional nucleic acid may have a primer site for a second primer.

In any embodiment, the secondary antibody is not detectably labeled (i.e., is "unlabeled"). In these embodiments, the secondary antibody is not conjugated to an enzyme (e.g., peroxidase or alkaline phosphatase), a fluorophore, a mass tag or an affinity agent such as biotin or streptavidin. In these embodiments, the oligonucleotide may be composed of unmodified nucleotides.

The species from which the primary and secondary antibodies are obtained may vary. In some embodiments, the primary antibody may be a rabbit or mouse monoclonal antibody, and the secondary antibody may an anti-mouse or anti-rabbit polyclonal antibody made in a different species to the primary antibody (e.g., goat). As with conventional secondary antibodies, the secondary antibody used in the present method can be a polyclonal antibody that has been raised against antibodies from the species of the primary antibody that has been affinity-purified or pre-adsorbed. In other embodiments, the secondary antibody may be an IgG fraction (or another multivalent fraction such as IgM) of the polyclonal antibody.

In any embodiment, the biological sample comprises cells. For example, the biological sample can be a cell suspension, a piece of tissue, or cells mounted on a microscope slide (e.g., an FFPE section). Sample include cultured cells that have been grown as a cell suspension, disassociated cells (which cells may have been produced by disassociating cultured cells or cells that are in a solid tissue, e.g., a soft tissue such as liver of spleen, using trypsin or the like). In particular embodiments, the complexes may be immobilized on cells that can be found in blood, e.g., cells that in whole blood or a subpopulation of cells thereof. Sub-populations of cells in whole blood include platelets, red blood cells (erythrocytes), and white blood cells (i.e., peripheral blood leukocytes, which are made up of neutrophils, lymphocytes, eosinophils, basophils and monocytes). These five types of white blood cells can be further divided into two groups, granulocytes (which are also known as polymorphonuclear leukocytes and include neutrophils, eosinophils and basophils) and mononuclear leukocytes (which include monocytes and lymphocytes). Lymphocytes can be further divided into T cells, B cells and NK cells. Peripheral blood cells are found in the circulating pool of blood and not sequestered within the lymphatic system, spleen, liver, or bone marrow. If cells that are immobilized on a support are used, then then the sample may be made by, e.g., growing cells on a planar surface, depositing cells on a planar surface, e.g., by centrifugation, by cutting a three dimensional object that contains cells into sections and mounting the sections onto a planar surface, i.e., producing a tissue section. In any embodiments, the cells may be fixed but not permeabilized.

In any embodiment, the cells used in the method may be free cells (i.e., cells that are not attached to a substrate or each other that in in a suspension, e.g., blood cells) that have been fixed (e.g., using a fixative such as PFA) but not permeabilized. Specifically, the cells may be blood cells that have been fixed using, e.g., PFA, but not treated with a detergent or any other permeabilization agent.

Examples of methods that the present method can be used in conjunction with include CODEX (Goltsev et al, Cell 2018 174, 968-981), in situ proximity ligation assays (PLA) (Bagchi et al Methods Mol Biol 2015 1318:149-59), a variety of barcoding methods in which the oligonucleotides are conjugated to a spatial barcode (i.e., a sequence that identifies a spatial location), released from the sample, and then sequenced (see, e.g., Kishi Nature Methods 2022 19: 1393-1402, among others), and molecular pixelation assays (which are described in greater detail below).

In some embodiments, the labeling step may be used in the context of a "molecular pixelation assay", as described in WO2022208327 and Karlsson et al (Molecular Pixelation: Single cell spatial proteomics by sequencing BioRxiv 2023.06.05.543770). A molecular pixelation assay is a DNA-sequencing based method for single cell analysis to quantify protein abundance, spatial distribution, and colocalization of targeted proteins using antibody-oligonucleotide conjugates. Relative locations of antibody-oligonucleotide conjugates are inferred by sequentially associating these into local neighborhoods using DNA-pixels containing unique pixel identifier (UPI) sequences, forming at least a thousand connected spatial zones per single cell in three dimensions. DNA-sequencing reads are computationally arranged into spatial single cell maps for multiple proteins without cell compartmentalization. By studying immune cell dynamics and using spatial statistics on graph representations of the data, new patterns of protein spatial polarization and colocalization can be identified.

Some of the principles of this method are described below where, in this description, the nucleic acid molecules that are bound to sites in or on cells are antibody-oligonucleotide conjugates. As would be apparent, the antibody-oligonucleotide conjugates can be bound to the sample in the presence of secondary antibody that recognizes the primary antibody of the primary antibody-oligonucleotide conjugates, thereby cross-linking the primary antibody and producing a cross-linked sample. In these embodiments, a molecular pixelation assay is for making a map of binding events in or on a cellular sample. In these embodiments, the assay may comprise: (a) obtaining: i. a sample containing nucleic acid molecules that are bound to sites in or on cells, the nucleic acid molecules having an extendible 5' or 3' end; ii. a first set of barcoded particles that each have a nucleotide sequence comprising in order: (i) a binding sequence that is complementary to the extendible end of the nucleic acid molecules of (a), (ii) a unique particle identifier sequence, and (iii) a first template sequence, wherein each particle can be distinguished from each other particle by their unique identifier sequences; iii. a second set of barcoded particles that each have a nucleotide sequence comprising: (i) the first template sequence, and (ii) a unique particle identifier sequence, wherein the nucleotide sequence of the second set of barcoded particles does not contain the binding sequence and wherein each particle can be distinguished from each other particle by their unique identifier sequences; (b) specifically hybridizing the first set of barcoded particles of (a)(ii) with the sample, wherein the nucleotide sequence of at least some of the first set of barcoded particles hybridizes to at least two of the nucleic acid molecules of (a); (c) extending the nucleic acid molecules that are hybridized to barcoded particles in step (b) using first barcoded particles as a template to produce first extension products that each comprise a first unique particle identifier sequence; (d) removing the first set of barcoded particles from the sample; (e) specifically hybridizing the second set of barcoded particles of (a)(iii) with the first extension products of (c), wherein the nucleotide sequences of at least some of the second set of barcoded particles hybridizes to at least two molecules of the first extension products; (f) extending the first extension products that are hybridized to a second barcoded particle in step (e) using the second barcoded particles as a template to produce second extension products that comprise the two unique particle identifier sequences; (g) determining which unique particle identifier sequence or complements thereof are in second extension products; and (h) making a map of the relative positions of the nucleic acid of (a)(i) using the unique particle identifier sequences that are in the second extension products. the sample of (a) is made by binding oligonucleotides that are linked to binding agents to sites that are in or on the cells. As ntoed above, the binding agents may be antibody-oligonucleotide conjugates and, in these embodiments, wherein the oligonucleotides may further comprise target identifier sequences that indicate the binding agent to which they are linked.

The following probe system may be used in the assay. In some embodiment, the probe system may comprise: (a) a population of nucleic acid molecules that have an extendible 5' or 3' end; (b) a first set of barcoded particles that each have a nucleotide sequence comprising in order: (i) a binding sequence that is complementary to the extendible end of the nucleic acid molecules of (a), (ii) a unique particle identifier sequence, and (iii) a first template sequence; wherein each particle can be distinguished from each other particle by their unique identifier sequences; (c) a second set of barcoded particles that each have a nucleotide sequence comprising: (i) the first template sequence, and (ii) a unique particle identifier sequence, wherein the nucleotide sequence of the second set of barcoded particles does not contain the binding sequence and wherein each particle can be distinguished from each other particle by their unique identifier sequences; wherein extension of the nucleic acid molecules of (a) using the first set of barcoded particles of (b) as a template produces first extension products that contain the complement of a unique particle identifier sequence of a particle of (b)(ii) and the complement of the first template sequence of the particles of the first set and of the second set; and wherein extension of the first extension products using the second set of barcoded particles as a template produces second extension products that contain the complement of a unique particle identifier sequence of a particle of (c)(ii), the complement of the first template sequence and the complement of a unique particle identifier sequence of a particle of (b)(ii). In some embodiments, the barcoded particles of (b) and/or (c) are rolling circle amplification (RCA) products.

### Utility

The methods and compositions described herein find general use in a wide variety of application for analysis of a sample (e.g., in the analysis of tissue sections, sheets of cells, spun-down cells, cell suspensions, blood cells, etc.).

In certain embodiments, one or more of the antibodies may specifically bind to biomarkers, including cancer biomarkers, that may be proteinaceous, e.g., cell surface markers. Exemplary cancer biomarkers, include, but are not limited to carcinoembryonic antigen (for identification of adenocarcinomas), cytokeratins (for identification of carcinomas but may also be expressed in some sarcomas), CD15 and CD30 (for Hodgkin's disease), alpha fetoprotein (for yolk sac tumors and hepatocellular carcinoma), CD117 (for gastrointestinal stromal tumors), CD10 (for renal cell carcinoma and acute lymphoblastic leukemia), prostate specific antigen (for prostate cancer), estrogens and progesterone (for tumour identification), CD20 (for identification of B-cell lymphomas) and CD3 (for identification of T-cell lymphomas).

The above-described method can be used to analyze cells from a subject to determine, for example, whether the cell is normal or not or to determine whether the cells are responding to a treatment. In one embodiment, the method may be employed to determine the degree of dysplasia in cancer cells. In these embodiments, the cells may be a sample from a multicellular organism.

The method described above finds particular utility in examining samples using a plurality of antibodies, each antibodies recognizing a different marker. Examples of cancers, and biomarkers that can be used to identify those cancers, are shown below. In these embodiments, one does not need to examine all of the markers listed below.

| | |
|---|---|
| Acute Leukemia IHC Panel | CD3, CD7, CD20, CD34, CD45, CD56, CD117, MPO, PAX-5, and TdT. |
| Adenocarcinoma vs. Mesothelioma IHC Panel | Pan-CK, CEA, MOC-31, BerEP4, TTF1, calretinin, and WT-1. |
| Bladder vs. Prostate Carcinoma IHC Panel | CK7, CK20, PSA, CK 903, and p63. |
| Breast IHC Panel | ER, PR, Ki-67, and HER2. Reflex to HER2 FISH after HER2 IHC is available. |
| Burkitt vs. DLBC Lymphoma IHC panel | BCL-2, c-MYC, Ki-67. |
| Carcinoma Unknown Primary Site, Female (CUPS IHC Panel - Female ) | CK7, CK20, mammaglobin, ER, TTF1, CEA, CA19-9, S100, synaptophysin, and WT-1. |
| Carcinoma Unknown Primary Site, Male (CUPS IHC Panel - Male) | CK7, CK20, TTF1, PSA, CEA, CA19-9, S100, and synaptophysin. |
| GIST IHC Panel | CD117, DOG-1, CD34, and desmin. |
| Hepatoma/Cholangio vs. Metastatic Carcinoma IHC Panel | HSA (HepPar 1), CDX2, CK7, CK20, CAM 5.2, TTF-1, and CEA (polyclonal). |
| Hodgkin vs. NHL IHC Panel | BOB-1, BCL-6, CD3, CD10, CD15, CD20, CD30, CD45 LCA, CD79a, MUM1, OCT-2, PAX-5, and EBER ISH. |
| Lung Cancer IHC Panel | chromogranin A, synaptophysin, CK7, p63, and TTF-1. |
| Lung vs. Metastatic Breast Carcinoma IHC Panel | TTF1, mammaglobin, GCDFP-15 (BRST-2), and ER. |
| Lymphoma Phenotype IHC Panel | BCL-2, BCL-6, CD3, CD4, CD5, CD7, CD8, CD10, CD15, CD20, CD30, CD79a, CD138, cyclin D1, Ki67, MUM1, PAX-5, TdT, and EBER ISH. |
| Lymphoma vs. Carcinoma IHC Panel | CD30, CD45, CD68, CD117, pan-keratin, MPO, S100, and synaptophysin. |
| Lymphoma vs. Reactive Hyperplasia IHC Panel | BCL-2, BCL-6, CD3, CD5, CD10, CD20, CD23, CD43, cyclin D1, and Ki-67. |
| Melanoma vs. Squamous Cell Carcinoma IHC Panel | CD68, Factor XIIIa, CEA (polyclonal), S-100, melanoma cocktail (HMB-45, MART-1/Melan-A, tyrosinase) and Pan-CK. |
| Mismatch Repair Proteins IHC Panel (MMR/Colon Cancer) | MLH1, MSH2, MSH6, and PMS2. |
| Neuroendocrine Neoplasm IHC Panel | CD56, synaptophysin, chromogranin A, TTF-1, Pan-CK, and CEA (polyclonal). |
| Plasma Cell Neoplasm IHC Panel | CD19, CD20, CD38, CD43, CD56, CD79a, CD138, cyclin D1, EMA, kappa, lambda, and MUM1. |
| Prostate vs. Colon Carcinoma IHC Panel | CDX2, CK 20, CEA (monoclonal), CA19-9, PLAP, CK 7, and PSA. |
| Soft Tissue Tumor IHC Panel | Pan-CK, SMA, desmin, S100, CD34, vimentin, and CD68. |
| T-Cell Lymphoma IHC panel | ALK1, CD2, CD3, CD4, CD5, CD7, CD8, CD10, CD20, CD21, CD30, CD56, TdT, and EBER ISH. |
| T-LGL Leukemia IHC panel | CD3, CD8, granzyme B, and TIA-1. |
| Undifferentiated Tumor IHC Panel | Pan-CK, S100, CD45, and vimentin. |

The compositions and methods described herein can be used to diagnose a patient with a disease. In some cases, the presence or absence of a biomarker in the patient's sample can indicate that the patient has a particular disease (e.g., cancer). In some cases, a patient can be diagnosed with a disease by comparing a sample from the patient with a sample from a healthy control. In this example, a level of a biomarker, relative to the control, can be measured. A difference in the level of a biomarker in the patient's sample relative to the control can be indicative of disease. In some cases, one or more biomarkers are analyzed in order to diagnose a patient with a disease. The compositions and methods of the disclosure are particularly suited to identifying the presence or absence of, or determining expression levels, of a plurality of biomarkers in a sample.

In some cases, the compositions and methods herein can be used to determine a treatment plan for a patient. The presence or absence of a biomarker may indicate that a patient is responsive to or refractory to a particular therapy. For example, a presence or absence of one or more biomarkers may indicate that a disease is refractory to a specific therapy and an alternative therapy can be administered. In some cases, a patient is currently receiving the therapy and the presence or absence of one or more biomarkers may indicate that the therapy is no longer effective.

In any embodiment, data can be forwarded to a "remote location", where "remote location," means a location other than the location at which the data produced by the method is examined. For example, a remote location could be another location (e.g., office, lab, etc.) in the same city, another location in a different city, another location in a different state, another location in a different country, etc. As such, when one item is indicated as being "remote" from another, what is meant is that the two items can be in the same room but separated, or at least in different rooms or different buildings, and can be at least one mile, ten miles, or at least one hundred miles apart. "Communicating" information references transmitting the data representing that information as electrical signals over a suitable communication channel (e.g., a private or public network). "Forwarding" an item refers to any means of getting that item from one location to the next, whether by physically transporting that item or otherwise (where that is possible) and includes, at least in the case of data, physically transporting a medium carrying the data or communicating the data. Examples of communicating media include radio or infra-red transmission channels as well as a network connection to another computer or networked device, and the internet or including email transmissions and information recorded on websites and the like. In certain embodiments, the data may be analyzed by an MD or other qualified medical professional, and a report based on the results of the analysis of the data may be forwarded to the patient from which the sample was obtained.

In some cases, the method may be employed in a variety of diagnostic, drug discovery, and research applications that include, but are not limited to, diagnosis or monitoring of a disease or condition (where the data identifies a marker for the disease or condition), discovery of drug targets (where the a marker in the data may be targeted for drug therapy), drug screening (where the effects of a drug are monitored by a marker shown in the data ), determining drug susceptibility (where drug susceptibility is associated with a marker) and basic research (where is it desirable to measure the differences between cells in a sample).

In certain embodiments, two different samples may be compared using the above methods. The different samples may be composed of an "experimental" sample, i.e., a sample of interest, and a "control" sample to which the experimental sample may be compared. In many embodiments, the different samples are pairs of cell types or fractions thereof, one cell type being a cell type of interest, e.g., an abnormal cell, and the other a control, e.g., normal, cell. If two fractions of cells are compared, the fractions are usually the same fraction from each of the two cells. In certain embodiments, however, two fractions of the same cell may be compared. Exemplary cell type pairs include, for example, cells isolated from a tissue biopsy (e.g., from a tissue having a disease such as colon, breast, prostate, lung, skin cancer, or infected with a pathogen etc.) and normal cells from the same tissue, usually from the same patient; cells grown in tissue culture that are immortal (e.g., cells with a proliferative mutation or an immortalizing transgene), infected with a pathogen, or treated (e.g., with environmental or chemical agents such as peptides, hormones, altered temperature, growth condition, physical stress, cellular transformation, etc.), and a normal cell (e.g., a cell that is otherwise identical to the experimental cell except that it is not immortal, infected, or treated, etc.); a cell isolated from a mammal with a cancer, a disease, a geriatric mammal, or a mammal exposed to a condition, and a cell from a mammal of the same species, preferably from the same family, that is healthy or young; and differentiated cells and non-differentiated cells from the same mammal (e.g., one cell being the progenitor of the other in a mammal, for example). In one embodiment, cells of different types, e.g., neuronal and non-neuronal cells, or cells of different status (e.g., before and after a stimulus on the cells) may be employed. In another embodiment of the invention, the experimental material is cells susceptible to infection by a pathogen such as a virus, e.g., human immunodeficiency virus (HIV), etc., and the control material is cells resistant to infection by the pathogen. In another embodiment, the sample pair is represented by undifferentiated cells, e.g., stem cells, and differentiated cells.

Cells any organism, e.g., from bacteria, yeast, plants and animals, such as fish, birds, reptiles, amphibians and mammals may be used in the subject methods. In certain embodiments, mammalian cells, i.e., cells from mice, rabbits, primates, or humans, or cultured derivatives thereof, may be used.

### EXAMPLES

The following examples are put forth so as to provide those of ordinary skill in the art with additional disclosure and description of how to make and use the present invention, and are not intended to limit the scope of what the inventors regard as their invention nor are they intended to represent that the experiments below are all or the only experiments performed.

### EXAMPLE 1

Raji B-cells were harvested from its culture media, washed and resuspended in PBS. Fc-repector blocking was performed by incubating the cells with Human Trustain FcX blocking solution (Biolegend). Cells were then fixated using 1% PFA solution for 15 minutes, followed by quenching of unreacted PFA and a series of washing steps. The fixed cells were stained with a pool of 20 antibody-oligonucleotide conjugates for 45 minutes at 4°C, followed by 3 washing steps to remove any unbound conjugates. Following binding of antibody-oligo conjugates and washing away unbound conjugates, a secondary antibody (goat anti mouse IgG) was added at a concentration of 5 ug/ml and incubated for 40 minutes at 37°C.

A molecular pixelation assay (see, e.g., WO2022208327, Karlsson et al (Molecular Pixelation: Single cell spatial proteomics by sequencing BioRxiv 2023.06.05.543770) the description of the same that can be found earlier in this disclosure) was performed on samples without or with the secondary antibody incubation to assess the ability of secondary antibody binding on stabilizing the primary antibodies bound to its target proteins on the cell surface. PCR was performed on the generated amplicons from the Molecular Pixelation assay and the signal strength was assessed using PAGE.

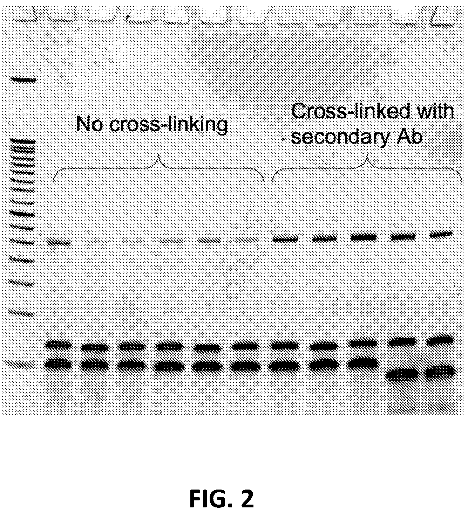

The signal strength on PAGE corresponds to the number of conjugate molecules on each cell at the point of performing the PCR. An increased signal strength seen by addition of the secondary antibody following binding of the primary antibodies thus indicates that the presence of the secondary antibody helped stabilize the conjugates and keep them bound to the cells throughout the molecular pixelation protocol. The signal strength was on average 4.7 times higher for samples prepared using a secondary antibody compared to samples without the secondary antibody addition. These results are shown in Fig. 2.

### EXAMPLE 2

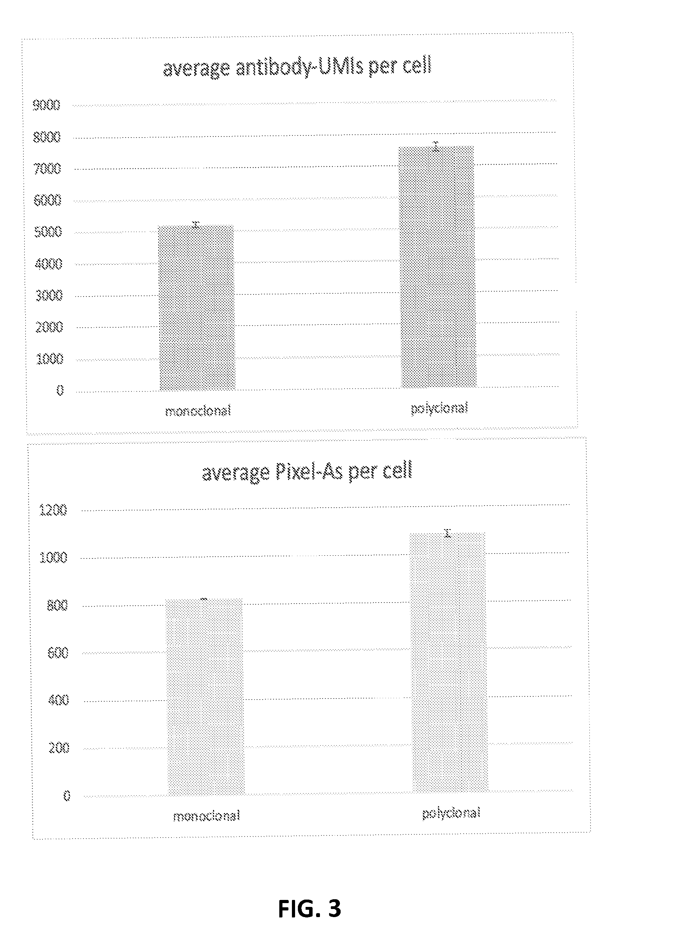

In this example, PFA fixed PBMCs were first stained with 80 target specific mouse monoclonal antibody-oligonucleotide conjugates (AOCs) for a molecular pixelation assay as described in Example 1. Two types of secondary antibodies were then used to cross-link the primary antibodies in place, either a monoclonal antibody (RM104 Abcam) or a polyclonal antibody (Goat anti-Mouse IgG Antibody Antibodies-online.com, ABIN2690354). The secondary antibody was allowed to bind for 60 minutes at 37 C before hybridization and gap-fill ligation by DNA-Pixel A and DNA-Pixel B copying the unique pixel identifier into the sequence of the AOC for spatial mapping of immune cell surface proteins. The sample was subjected to PCR for sequencing preparation. The number of unique reads per cell was counted from the sequencing reads as well as the number of DNA-Pixel As per cell, Fig. 3 shows the results with standard deviations of two replicates.

As shown in Fig. 3, the polyclonal secondary antibody cross-links the primary AOCs more efficiently compared to the monoclonal secondary as both more antibody-UMIs per cell and Pixel-As per cell are preserved using the polyclonal secondary. This may due to the polyclonal antibody binding to more sites on the primary AOC than a monoclonal, providing more efficient cross-linking.

## Claims

1. A method for labeling a biological sample comprising:
(a) labeling a biological sample that has been crosslinked by a fixative with one or more primary antibody-oligonucleotide conjugates to produce a labeled sample, wherein the biological sample comprises cells;
(b) incubating the labeled sample with a secondary antibody that recognizes the primary antibody of the primary antibody-oligonucleotide conjugates, thereby cross-linking the primary antibody and producing a cross-linked sample;
(c) performing one or more molecular reactions on the oligonucleotide of the primary antibody-oligonucleotide conjugates to produce a nucleic acid product, wherein the molecular reaction comprises:
a hybridization, ligation, primer extension, or gap fill, or any combination thereof,
a hybridization with a nucleic acid followed by a primer extension and/or gap-fill reaction using the oligonucleotide or the nucleic acid as a template,
a splinted ligation to a nucleic acid, or
a proximity assay; and
(d) detecting the nucleic acid product of (c).

2. The method of claim 1, wherein the molecular reactions of (c) comprise exposing the primary antibody-oligonucleotide conjugates to one or more disassociating conditions while they are bound to the sample, wherein the disassociating conditions comprise one or more reactions, hybridizations, washes and/or treatments in at least 150 mM salt or an equivalent thereof at a temperature of at least 37 °C for at least 1 minute.

3. The method of claim 1 or 2, wherein the molecular reactions of (c) comprise exposing the primary antibody-oligonucleotide conjugates a physical shearing stress.

4. The method of claim 3, wherein the physical searing stress is by tethering to particle selected from a rolling circle amplification product or bead.

5. The method of any prior claim, wherein the oligonucleotide of a conjugate comprises an antibody identifier sequence that uniquely identifies the primary antibody to which it is conjugated.

6. The method of claim 5, wherein:
step (a) comprises *en masse* labeling the biological sample with a plurality of primary antibody-oligonucleotide conjugates that recognize different targets in or on the sample; and
step (d) comprises sequencing at least the antibody identifier sequences in the nucleic acid reaction product of (c).

7. The method of any prior claim, wherein the molecular reaction of (c) is a hybridization, ligation, primer extension, or gap fill, or any combination thereof.

8. The method of any prior claim, wherein the molecular reaction of (c) comprises a hybridization with a nucleic acid followed by a primer extension and/or gap-fill reaction using the oligonucleotide or the nucleic acid as a template.

9. The method of any of claims 1-7, wherein the molecular reaction of (c) is a splinted ligation to a nucleic acid.

10. The method of any prior claim, wherein the molecular reaction of (c) is a proximity assay.

11. The method of any prior claim, wherein the detecting of (d) is done by sequencing the product, or an amplification product thereof.

12. The method of any prior claim, wherein the secondary antibody is unlabeled.

13. The method of any prior claim, wherein the primary antibody is a rabbit or mouse monoclonal antibody, and the secondary antibody is an anti-mouse or anti-rabbit polyclonal antibody made in a different species to the primary antibody.

14. The method of claim 13, wherein the different species is goat.

15. The method of any prior claim, wherein the secondary antibody is an affinity-purified or pre-adsorbed polyclonal antibody, or an IgG fraction of a polyclonal antibody.

16. The method any prior claim, wherein the biological sample is a cell suspension, a piece of tissue, or a tissue section.

17. The method of any prior claim, wherein step (c) is done in the presence of additional secondary antibodies.

## Patentansprüche

1. Verfahren zum Markieren einer biologischen Probe, aufweisend:
(a) Markieren einer biologischen Probe, die durch ein Fixiermittel vemetzt wurde, mit einem oder mehreren primären Antikörper-Oligonukleotid-Konjugaten, um eine markierte Probe herzustellen, wobei die biologische Probe Zellen aufweist;
(b) Inkubieren der markierten Probe mit einem sekundären Antikörper, der den primären Antikörper der primären Antikörper-Oligonukleotid-Konjugate erkennt, wodurch der primäre Antikörper vernetzt wird und eine vernetzte Probe hergestellt wird;
(c) Durchführen einer oder mehrerer molekularer Reaktionen an dem Oligonukleotid der primären Antikörper-Oligonukleotid-Konjugate, um ein Nukleinsäureprodukt herzustellen, wobei die molekulare Reaktion aufweist:
eine Hybridisierung, Ligation, Primerverlängerung oder Lückenfüllung oder eine beliebige Kombination hiervon,
eine Hybridisierung mit einer Nukleinsäure, gefolgt von einer Primerverlängerung und/oder einer Lückenfüllungsreaktion unter Verwendung des Oligonukleotids oder der Nukleinsäure als Vorlage,
eine gesplintete Ligation an eine Nukleinsäure oder
einen Annäherungsassay; und
(d) Nachweisen des Nukleinsäureprodukts aus (c).

2. Verfahren gemäß Anspruch 1, wobei die molekularen Reaktionen von (c) das Aussetzen der primären Antikörper-Oligonukleotid-Konjugate einer oder mehreren Dissoziationsbedingungen aufweisen, während sie an die Probe gebunden sind, wobei die Dissoziationsbedingungen eine oder mehrere Reaktionen, Hybridisierungen, Waschvorgänge und/oder Behandlungen in mindestens 150 mM Salz oder einem Äquivalent davon bei einer Temperatur von mindestens 37 °C für mindestens 1 Minute aufweisen.

3. Verfahren gemäß Anspruch 1 oder 2, wobei die molekularen Reaktionen von (c) das Aussetzen der primären Antikörper-Oligonukleotid-Konjugate einer physikalischen Scherbeanspruchung aufweisen.

4. Verfahren gemäß Anspruch 3, wobei die physikalische Scherbeanspruchung durch Anbinden an ein Partikel erfolgt, das aus einem Rolling-Circle-Amplifikationsprodukt oder einem Kügelchen ausgewählt ist.

5. Verfahren gemäß einem der vorangehenden Ansprüche, wobei das Oligonukleotid eines Konjugats eine Antikörper-Identifizierungssequenz aufweist, die den primären Antikörper, an den es konjugiert ist, eindeutig identifiziert.

6. Verfahren gemäß Anspruch 5, wobei:
Schritt (a) das Massenmarkieren der biologischen Probe mit mehreren Primärantikörper-Oligonukleotid-Konjugaten aufweist, die verschiedene Ziele in oder auf der Probe erkennen;
und Schritt (d) das Sequenzieren mindestens der Antikörper-Identifikationssequenzen im Nukleinsäure-Reaktionsprodukt von (c) aufweist.

7. Verfahren gemäß einem der vorangehenden Ansprüche, wobei die molekulare Reaktion von (c) eine Hybridisierung, Ligation, Primerverlängerung oder Lückenfüllung oder eine beliebige Kombination hiervon ist.

8. Verfahren gemäß einem der vorangehenden Ansprüche, wobei die molekulare Reaktion von (c) eine Hybridisierung mit einer Nukleinsäure aufweist, gefolgt von einer Primerverlängerung und/oder einer Lückenfüllungsreaktion unter Verwendung des Oligonukleotids oder der Nukleinsäure als Vorlage.

9. Verfahren gemäß einem der Ansprüche 1-7, wobei die molekulare Reaktion von (c) eine gesplintete Ligation an eine Nukleinsäure ist.

10. Verfahren gemäß einem der vorhergehenden Ansprüche, wobei die molekulare Reaktion von (c) ein Annäherungsassay ist.

11. Verfahren gemäß einem der vorangehenden Ansprüche, wobei das Nachweisen nach (d) durch Sequenzieren des Produkts oder eines Amplifikationsprodukts hiervon erfolgt.

12. Verfahren gemäß einem der vorangehenden Ansprüche, wobei der sekundäre Antikörper unmarkiert ist.

13. Verfahren gemäß einem der vorangehenden Ansprüche, wobei der primäre Antikörper ein monoklonaler Antikörper von Kaninchen oder Maus ist, und der sekundäre Antikörper ein polyklonaler Anti-Maus- oder Anti-Kaninchen-Antikörper ist, der in einer anderen Spezies als der primäre Antikörper erzeugt wurde.

14. Verfahren gemäß Anspruch 13, wobei die andere Spezies Ziege ist.

15. Verfahren gemäß einem der vorangehenden Ansprüche, wobei der sekundäre Antikörper ein affinitätsgereinigter oder voradsorbierter polyklonaler Antikörper oder eine IgG-Fraktion eines polyklonalen Antikörpers ist.

16. Verfahren gemäß einem der vorangehenden Ansprüche, wobei die biologische Probe eine Zellsuspension, ein Gewebestück oder ein Gewebeschnitt ist.

17. Verfahren gemäß einem der vorangehenden Ansprüche, wobei Schritt (c) in Gegenwart zusätzlicher sekundärer Antikörper durchgeführt wird.

## Revendications

1. Procédé de marquage d'un échantillon biologique comprenant :
(a) Marquer l'échantillon biologique qui a été réticulé par un fixateur avec un ou plusieurs conjugués anticorps primaire - oligonucléotide afin de produire un échantillon marqué, dans lequel l'échantillon biologique comprend des cellules ;
(b) Incuber l'échantillon marqué avec un anticorps secondaire qui reconnait l'anticorps primaire des conjugués anticorps primaire - oligonucléotide, réticulant ainsi l'anticorps primaire et produisant un échantillon réticulé ;
(c) Réaliser une ou plusieurs réactions moléculaires sur l'oligonucléotide des conjugués anticorps primaire - oligonucléotide pour produire un produit d'acide nucléique, dans lequel la réaction moléculaire comprend :
Une hybridation, ligation, extension d'amorce ou un remplissage d'espace, ou
toute combinaison de ceux-ci,
Une hybridation avec un acide nucléique suivie d'une extension d'amorce et/ou une réaction de remplissage d'espace en utilisant l'oligonucléotide ou l'acide nucléique comme matrice,
Une ligature pontée à un acide nucléique, ou
Un test de proximité ; et
(d) Détecter le produit d'acide nucléique de (c).

2. Le procédé selon la revendication 1, dans lequel les réactions moléculaires de (c) comprennent l'exposition des conjugués anticorps primaire - oligonucléotide à une ou plusieurs conditions de dissociation alors qu'ils sont liés à l'échantillon, dans lequel les conditions de dissociation comprennent une ou plusieurs réactions, hybridations, lavages et/ou traitements dans au moins 150mM de sel ou un équivalent de celui-ci à une température d'au moins 37°C pendant au moins 1 minute.

3. Le procédé selon la revendication 1 ou 2, dans lequel les réactions moléculaires de (c) comprennent l'exposition des conjugués anticorps primaire - oligonucléotide à une contrainte de cisaillement physique.

4. Le procédé selon la revendication 3, dans lequel la contrainte physique de cisaillement physique est exercée par attache à une particule sélectionnée à partir d'une bille ou produit d'amplification à cercle roulant.

5. Le procédé selon une des revendications précédentes, dans lequel l'oligonucléotide d'un conjugué comprend une séquence d'identification d'anticorps qui identifie de manière unique l'anticorps primaire auquel il est conjugué.

6. Le procédé selon la revendication 5, dans lequel :
L'étape (a) comprend le marquage en masse de l'échantillon biologique avec une pluralité de conjugués anticorps primaire - oligonucléotide qui reconnait différentes cibles dans ou sur l'échantillon ;
L'étape (d) comprend le séquençage des séquences d'identification d'au moins un anticorps dans le produit de réaction d'acide nucléique de (c).

7. Le procédé selon une des revendications précédentes, dans lequel la réaction moléculaire de (c) est une hybridation, une ligation, une extension d'amorce, un remplissage d'espace, ou toute combinaison de celles-ci.

8. Le procédé selon une des revendications précédentes, dans lequel la réaction moléculaire de (c) comprend une hybridation avec un acide nucléique suivie d'une extension d'amorce et/ou une réaction remplissage d'espace en utilisant l'oligonucléotide ou l'acide nucléique comme matrice.

9. Le procédé selon une des revendications 1-7, dans lequel la réaction moléculaire de (c) est une ligature pontée à un acide nucléique.

10. Le procédé selon une des revendications précédentes, dans lequel la réaction moléculaire de (c) est un test de proximité.

11. Le procédé selon une des revendications précédentes, dans lequel la détection de (d) est effectuée par séquençage du produit, ou d'un produit d'amplification dudit produit.

12. Le procédé selon une des revendications précédentes, dans lequel l'anticorps secondaire n'est pas marqué.

13. Le procédé selon une des revendications précédentes, dans lequel l'anticorps primaire est un anticorps monoclonal de lapin ou de souris, et l'anticorps secondaire est un anticorps polyclonal anti-souris ou anti-lapin produit chez une espèce différente de celle de l'anticorps primaire.

14. Le procédé de la revendication 13, dans lequel l'espèce différente est la chèvre.

15. Le procédé selon une des revendications précédentes, dans lequel l'anticorps secondaire est un anticorps polyclonal purifié par affinité ou pré-adsorbé, ou une fraction IgG d'un anticorps polyclonal.

16. Le procédé selon une des revendications précédentes, dans lequel l'échantillon biologique est une suspension cellulaire, un morceau de tissu, ou une section de tissu.

17. Le procédé selon une des revendications précédentes, dans lequel l'étape (c) est effectuée en présence d'anticorps secondaires supplémentaires.
